# EUROPEAN PATENT APPLICATION

(11) **EP 4 212 184 A1**
(43) Date of publication of application: **19.07.2023**
(21) Application number: 21866330.0
(22) Date of filing: 22.06.2021
(51) Int. Cl.: A61L 2/10, A61L 2/24, A61L 9/20

(54) **STERILIZATION SYSTEM, STERILIZATION DEVICE, CONTROL DEVICE, CONTROL METHOD, AND CONTROL PROGRAM**

(30) Priority: 14.09.2020 JP 2020153591
(71) Applicant: Minebea Mitsumi Inc., Kitasaku-gun, Nagano 3890293 (JP)
(72) Inventor: IMAMURA, Shogo, Kitasaku-gun, Nagano 389-0293 (JP); SAGAWA, Yoshihiro, Kitasaku-gun, Nagano 389-0293 (JP)
(74) Representative: Zabel, Julia Elisabeth
(86) International application number: PCT/JP2021/023612
(87) International publication number: WO 2022/054369

(57) **Abstract**

A sterilization system of an embodiment includes a light source unit (21), a horizontal angle adjustment unit (4) and/or a vertical angle adjustment unit (3), and a controller (5). The light source unit (21) emits ultraviolet rays in a radiating form. The horizontal angle adjustment unit (4) adjusts a horizontal angle of the light source unit (21) by causing rotation of the light source unit (21) in a horizontal direction. The vertical angle adjustment unit (3) adjusts a vertical angle of the light source unit (21) by causing rotation of the light source unit (21) in a vertical direction. The controller (5) receives a marker signal transmitted from a marker (9) via a marker sensor (6), controls rotation of the horizontal angle adjustment unit (4) and/or the vertical angle adjustment unit (3) to direct an optical axis of the light source unit (21) to the marker (9), and stores a horizontal angle and/or a vertical angle of the light source unit (21) in response to a position setting request.

## Description

### Technical Field

The present invention relates to a sterilization system, a sterilizer, a controller, a control method, and a control program.

### Background Art

A sterilizer using ultraviolet ray (ultraviolet light) irradiation to sterilize bacteria without affecting human cells has been proposed and known (see, for example, Patent Document 1 and the like).

### Citation List

### Patent Literature

Patent Document 1: JP 6025756 B

### Summary of Invention

### Technical Problem

However, the known sterilizer performs sterilization on a certain target object to be sterilized, and does not correspond to sterilization for a wide range of space such as a room and factory.

On the other hand, sterilization target objects such as viruses, including COVID-19, and bacteria float in a space and adhere to a wall forming the space and the like. For the reason, there has been no choice but to take measures, and the implementation of the measures is limited due to the necessity of changing air conditioning equipment or due to a large workload, such as replacement of air in the space or inactivation by spraying an antiseptic solution. There has been a demand for easier improvement of the environment in the space.

The present invention has been made in view of the above, and an object of the present invention is to provide a sterilization system, a sterilizer, a controller, a control method, and a control program capable of easily sterilizing a wide range of sterilization target space.

### Solution to Problem

A sterilizer according to an aspect of the present invention to solve the problem described above and achieve the object includes a light source unit, a horizontal angle adjustment unit and/or a vertical angle adjustment unit, and a controller. The light source unit emits ultraviolet rays in a radiating form. The horizontal angle adjustment unit adjusts a horizontal angle of the light source unit by causing rotation of the light source unit in a horizontal direction. The vertical angle adjustment unit adjusts a vertical angle of the light source unit by causing rotation of the light source unit in a vertical direction. The controller receives a marker signal transmitted from a marker via a marker sensor, controls rotation of the horizontal angle adjustment unit and/or the vertical angle adjustment unit to direct an optical axis of the light source unit to the marker, and stores a horizontal angle and/or a vertical angle of the light source unit in response to a position setting request.

A sterilization system according to an aspect of the present invention can easily sterilize a wide range of sterilization target space.

### Brief Description of Drawings

FIG. 1 is a block diagram illustrating an example of a configuration of a sterilizer and the like according to an embodiment.
FIG. 2 is a diagram illustrating an example of a configuration in a case where a plurality of sterilizers are included.
FIG. 3 is a perspective view illustrating an example of a mechanical configuration of an example of the sterilizer.
FIG. 4 is a perspective view illustrating another example of a mechanical configuration of an example of the sterilizer.
FIG. 5 is a diagram illustrating an example of a hardware configuration of a control unit of a controller, a control unit of a terminal, or a control unit of a controller.
FIG. 6 is a diagram illustrating an example of a functional configuration of the control unit of the controller, the control unit of the terminal, or the control unit of the controller.
FIG. 7 is a flowchart illustrating an example of control by the control unit of the controller, the control unit of the terminal, or the control unit of the controller, using a marker.
FIG. 8 is a flowchart illustrating another example of control by the control unit of the controller, the control unit of the terminal, or the control unit of the controller, using the marker.
FIG. 9 is a flowchart illustrating an example of processing of calculating lifetime by the control unit of the controller, the control unit of the terminal, or the control unit of the controller.
FIG. 10 is a flowchart illustrating an example of processing of acquiring error information by the control unit of the controller, the control unit of the terminal, or the control unit of the controller.
FIG. 11 is a flowchart illustrating an example of processing of displaying the lifetime or the error information by the control unit of the controller, the control unit of the terminal, or the control unit of the controller.
FIG. 12 is a diagram illustrating an example of a display unit displaying the lifetime or the error information.

### Description of Embodiments

A sterilization system, a sterilizer, a controller, a control method, and a control program according to an embodiment will be described below with reference to the drawings. Note that the present invention is not limited to the embodiment described above. Furthermore, the dimensional relationships between elements, proportions of the elements, and the like in the drawings may differ from reality. The drawings may each include parts having mutually different dimensional relationships and proportions. Furthermore, the contents described in one embodiment or modified example are applied in principle to other embodiments or modified examples.

### Device Configuration, System Configuration

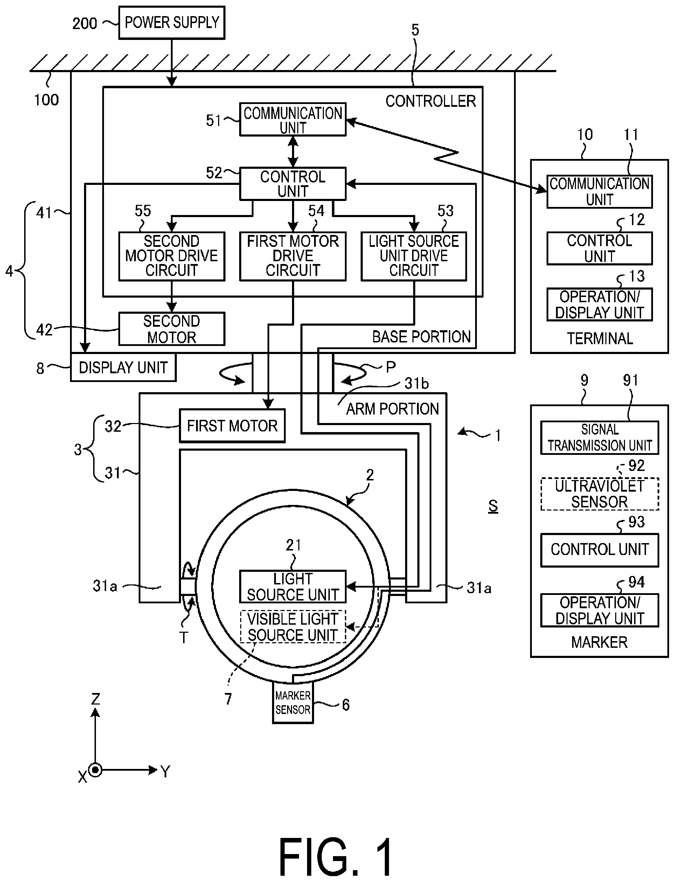

FIG. 1 is a block diagram illustrating an example of a configuration of a sterilizer 1 and the like according to an embodiment. In FIG. 1, the sterilizer 1 has a base portion 41 attached to a ceiling wall 100 forming a ceiling, for example, and includes: a main body portion 2 having a light source unit 21; a vertical angle adjustment unit 3 and a horizontal angle adjustment unit 4 rotatably supporting the main body portion 2; and a controller 5.

An X direction in FIG. 1 is a horizontal direction and is a front and back direction of the sterilizer 1A. A Y direction is a horizontal direction and is a width direction of the sterilizer 1A. A Z direction is a vertical direction orthogonal to the horizontal direction and is an upward and downward direction of the sterilizer 1A. In the present embodiment, the Z direction is parallel to the vertical direction of a sterilization target space S, but is not limited to the vertical direction, and the Z direction may be inclined with respect to the vertical direction or the Z direction may be parallel to a direction orthogonal to the vertical direction.

The controller 5 controls the sterilizer 1. The controller 5 controls the light source unit 21, the vertical angle adjustment unit 3, and the horizontal angle adjustment unit 4, and is contained in an internal space of the base portion 41. The controller 5 includes a communication unit 51, a control unit 52, a light source unit drive circuit 53, a first motor drive circuit 54, and a second motor drive circuit 55. Based on electric power supplied from a power supply 200, the controller 5 performs turning ON/OFF of emission of ultraviolet rays by the light source unit 21, adjustment of the irradiation intensity, adjustment of a vertical angle of the light source unit 21 by the vertical angle adjustment unit 3, adjustment of a horizontal angle of the light source unit 21 by the horizontal angle adjustment unit 4, and the like. The hardware configuration of the controller 5 is the same as the hardware configuration of a general controller, and will be described in detail below. The power supply 200 is a commercial power supply, a generator, a battery, or the like.

The communication unit 51 transmits and receives information to and from the terminal 10 carried by a user, and, for example, receives operation information from the terminal 10, and transmits a status of the sterilizer 1, an irradiation status (for example, ON/OFF, irradiation intensity) of the light source unit 21, and an orientation status (for example, the vertical angle and the horizontal angle) of the light source unit 21 to the terminal 10. The communication unit 51 performs short-range wireless communications using Wi-Fi (registered trademark), Bluetooth (registered trademark), or the like for example.

The control unit 52 performs irradiation control on the light source unit 21, drive control on a first motor 32, drive control on a second motor 42, and the like. Here, the irradiation control includes ON/OFF control and irradiation intensity control on the light source unit 21.
The light source unit drive circuit 53 supplies electric power to the light source unit 21 based on an irradiation control signal for the light source unit 21 from the control unit 52. When the light source unit 21 is of a type capable of changing the irradiation intensity of ultraviolet rays, the light source unit drive circuit 53 also adjusts the irradiation intensity. In addition, when the light source unit drive circuit 53 is of a type capable of changing (switching) the wavelength of the ultraviolet rays emitted by the light source unit 21, the light source unit drive circuit 53 also changes (switches) the wavelength. The light source unit drive circuit 53 is electrically connected to the control unit 52 and the light source unit 21. The light source unit drive circuit 53 is included in the controller 5, but is not limited to being included in the controller 5, and may be included in the light source unit 21. When the light source unit 21 is a light source combined with light sources of different wavelength ranges, adjustment is performed so that the irradiation intensity of each of the light sources can be adjusted.

The first motor drive circuit 54 supplies driving electric power to the first motor 32 based on a drive control signal for the first motor 32 from the control unit 52, to rotate the light source unit 21 in the vertical direction. The first motor drive circuit 54 is electrically connected to the control unit 52 and the first motor 32. The first motor drive circuit 54 is included in the controller 5 but is not limited to being included in the controller 5, and may be included in the first motor 32.

The second motor drive circuit 55 supplies driving electric power to the second motor 42 based on a drive control signal for the second motor 42 from the control unit 52, to rotate the light source unit 21 in the horizontal direction. The second motor drive circuit 55 is electrically connected to the control unit 52 and the second motor 42. The second motor drive circuit 55 is included in the controller 5 but is not limited to being included in the controller 5, and may be included in the second motor 42.

The user operates the terminal 10 to operate the sterilizer 1A, and to recognize the status of the sterilizer 1A. The terminal 10 includes a communication unit 11, a control unit 12, and an operation/display unit 13. The terminal 10 may be a dedicated terminal for the sterilizer 1A, or may be a general-purpose terminal such as a smartphone, tablet, and laptop computer. The hardware configuration of the terminal 10 is similar to the hardware configuration of a general terminal, and will be described in detail below.

The communication unit 11 transmits and receives information to and from the controller 5. The communication unit 11 performs short-range wireless communications using Wi-Fi (registered trademark), Bluetooth (registered trademark), or the like for example. Note that transmission and reception of information is not limited to wireless, and may be wired transmission and reception.

The control unit 12 controls the terminal 10 and outputs operation information to the communication unit 11 based on at least an operation on the operation/display unit 13 by the user. The control unit 12 also controls display by the operation/display unit 13. The control unit 12 is electrically connected to the communication unit 11 and the operation/display unit 13. Note that the control unit 12 can take over part of processing of the control unit 52 connected via the communication unit 11 on the terminal side and the communication unit 51 on the sterilizer 1 side.

The operation/display unit 13 is used for a remote operation of the sterilizer 1A in addition to a general operation and display in the terminal 10. Although not illustrated, the operation/display unit 13 includes, for example, a switch corresponding to turning ON/OFF of the ultraviolet rays, a switch corresponding to the irradiation intensity, a switch corresponding to adjustment of the vertical angle of the light source unit 21, and a switch corresponding to adjustment of the horizontal angle of the light source unit 21. Note that the switches may be a mechanical switch such as an energized/de-energized switch or a resistance change switch, or may be an electrical switch such as a touch panel.

A marker sensor 6 such as a complementary metal oxide semiconductor (CMOS) sensor is provided at the vicinity of the main body portion 2. The marker sensor 6 detects a marker signal based on infrared rays (infrared light) or the like emitted from a marker 9 on a two dimensional image. The control unit 52 recognizes the position of the marker 9 based on a detection signal (image signal) of the marker sensor 6, directs the irradiation direction of the ultraviolet rays to the marker 9, and sets the position and range to be irradiated with the ultraviolet rays for sterilization.

The marker 9 includes a signal transmission unit 91, an ultraviolet sensor 92, a control unit 93, and an operation/display unit 94. The ultraviolet sensor 92 may be omitted. The signal transmission unit 91 has a function of transmitting the marker signal by infrared rays, for example. When there are a plurality of markers 9, the marker signal transmitted from each marker 9 can be identified by changing the pulse pattern of the marker signal.

The ultraviolet sensor 92 has a function of detecting the ultraviolet rays emitted from the light source unit 21 of the sterilizer 1. Thus, the marker 9 being within the irradiation range of the ultraviolet ray irradiated by the sterilizer 1 can be recognized.

The control unit 93 controls the marker 9, and performs control for transmission of the marker signal by the signal transmission unit 91, confirmation of detection of ultraviolet rays by the ultraviolet sensor 92, and the like. The operation/display unit 94 receives an operation by the user and performs displaying of a status for the user and the like.

The main body portion 2 of the sterilizer 1 is provided with a visible light source unit 7 emitting visible light in parallel to the ultraviolet ray irradiation direction of the light source unit 21. The control unit 52 controls turning ON/OFF of the visible light source unit 7 via the light source unit drive circuit 53.

The base portion 41 and the like is provided with a display unit 8 such as a 7-segment LED display, and the display is controlled by the control unit 52. The contents of display by the display unit 8 include the lifetime of the light source unit 21 and error information on the sterilizer 1.

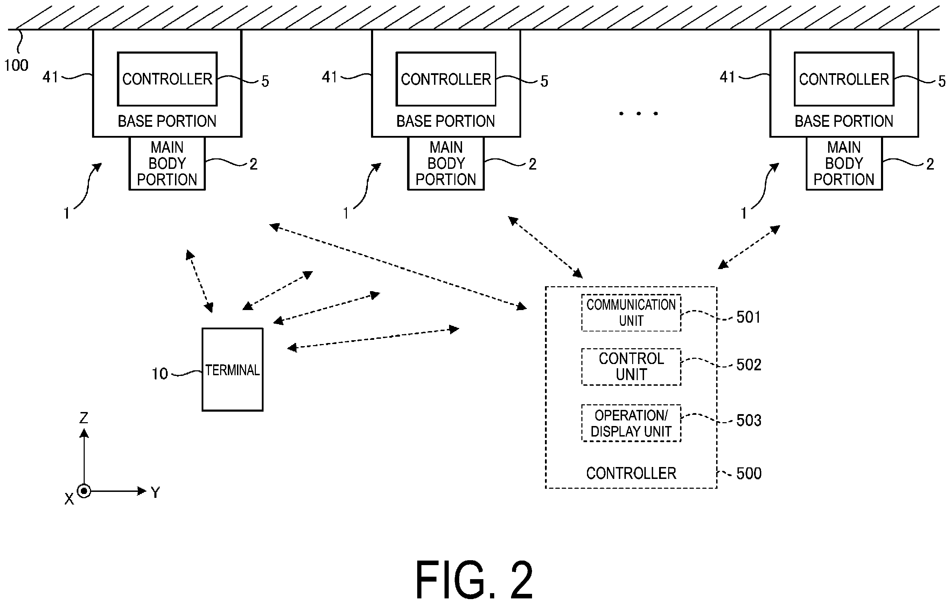

FIG. 2 is a diagram illustrating an example of a configuration in a case where a plurality of the sterilizers 1 are included. In FIG. 2, the plurality of sterilizers 1 are attached to the ceiling wall 100 forming a ceiling, for example. The controllers 5 of the respective sterilizers 1 perform control in an equal relationship, or one of the controllers 5 serves as a master to perform overall control. A controller 500 for the overall control may be provided separately from the controllers 5 of the sterilizers 1. The controller 500 includes a communication unit 501, a control unit 502, and an operation/display unit 503.

The communication unit 501 transmits and receives information to and from the controller 5 of each of the sterilizers 1. The communication unit 501 performs short-range wireless communications using Wi-Fi (registered trademark), Bluetooth (registered trademark), or the like for example. Note that transmission and reception of information is not limited to wireless, and may be wired transmission and reception.

The control unit 502 controls the controller 500 and outputs operation information to the communication unit 501 based on at least an operation on the operation/display unit 503 by the user or information set in advance. The control unit 502 is electrically connected to the communication unit 501 and the operation/display unit 503.

The operation/display unit 503 is used for a remote operation of each of the sterilizers 1 in addition to a general operation and display in the controller 500. Although not illustrated, the operation/display unit 503 includes, for example, a switch corresponding to turning ON/OFF of the ultraviolet rays, a switch corresponding to the irradiation intensity, a switch corresponding to adjustment of the vertical angle of the light source unit 21, a switch corresponding to adjustment of the horizontal angle of the light source unit 21, and a switch for setting various automatic operations. Note that the switches may be a mechanical switch such as an energized/de-energized switch or a resistance change switch, or may be an electrical switch such as a touch panel.

The terminal 10 communicates with the controller 5 of each of the sterilizers 1, with the controller 5 set to be the master, or with the controller 500, to correspond to various operations and status confirmation by the user. The terminal 10 can control each sterilizer 1 together with the controller 500 or in place of the controller 500.

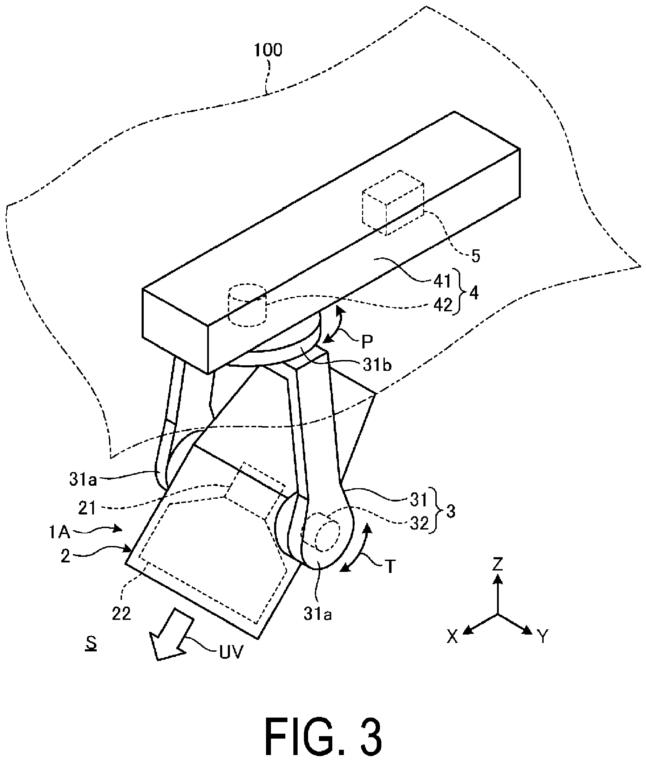

FIG. 3 is a perspective view illustrating an example of a mechanical configuration of an example of the sterilizer 1 (sterilizer 1A). As illustrated in FIG. 3, the sterilizer 1A irradiates the sterilization target space S with ultraviolet rays UV. The sterilizer 1A is attached to a wall forming a part of the sterilization target space S, that is, the ceiling wall 100 forming the ceiling in the present embodiment. Specifically, the sterilizer 1A is attached to the ceiling wall 100 via a rail or the like not illustrated, in a state of protruding from the ceiling wall 100 into the sterilization target space S. The sterilizer 1A includes the main body portion 2 with the light source unit 21 attached, the vertical angle adjustment unit 3, the horizontal angle adjustment unit 4, and the controller 5.

The light source unit 21 is attached to the main body portion 2. The main body portion 2 is formed in a substantially cylindrical shape, with an opening 22 formed at the sterilization target space S side in an axial direction and with the side opposite to the sterilization target space S closed. The main body portion 2 is supported, via a supporting shaft not illustrated, to be rotatable with respect to the vertical angle adjustment unit 3.

The light source unit 21 irradiates the sterilization target space S with the ultraviolet rays UV in a radiating form. The light source unit 21 is attached to a position opposing the opening 22 in the axial direction of the main body portion 2, in the internal space of the main body portion 2. The light source unit 21 irradiates the sterilization target space S with the ultraviolet rays UV through the opening 22. The ultraviolet rays UV emitted to the sterilization target space S by the light source unit 21 are electromagnetic waves having wavelengths different from the wavelength of visible light, and refer to electromagnetic waves having wavelengths of 190 nm to 230 nm in the present embodiment. Ultraviolet rays below 190 nm are absorbed by air, particularly oxygen in air. Thus, the amount of the ultraviolet rays reaching an object present in the sterilization target space S, for example, a human body, is greatly reduced, and the sterilization effect on the object is reduced. On the other hand, when the object present in the sterilization target space S is a human body, ultraviolet rays exceeding 230 nm may be absorbed by cells and damage DNA in the cells. The light source unit 21 of the present embodiment has, as the light source, a KrCl excimer lamp having a peak at the 222 nm for example. The ultraviolet rays UV in a radiating form may be emitted from the light source, or the ultraviolet rays of a radiating form may be obtained by a lens not illustrated from the ultraviolet rays UV in a linear form emitted from the light source.

The light source unit 21 is not limited to a light source emitting the ultraviolet rays UV in one wavelength range, and may be capable of switching between ultraviolet rays UV in different wavelength ranges. The light source unit 21 may also be a combination of a light source emitting ultraviolet rays in one wavelength range, and a light source emitting ultraviolet rays in a wavelength range different from the wavelength range. Specifically, the light source unit 21 may be a combination of a light source emitting ultraviolet rays having a wavelength of 190 nm to 230 nm, and a light source emitting ultraviolet rays having a wavelength of longer than 230 nm.

The vertical angle adjustment unit 3 rotates the light source unit 21 in the vertical direction Z, to adjust the vertical angle, that is, a tilt direction T. The vertical angle adjustment unit 3 includes an arm portion 31 and a first motor 32. The main body portion 2 is supported by the arm portion 31 to be rotatable in the vertical direction Z. The arm portion 31 is formed in a U-shape opening in the downward direction, and the main body portion 2 is supported, at both distal end portions 31a and 31a forming the opening, to be rotatable about a direction, and in the direction, the both distal end portions 31a and 31a oppose each other. The first motor 32 is a vertical actuator, and rotates the main body portion 2 with respect to the arm portion 31. The first motor 32 is attached to the arm portion 31 and rotates the main body portion 2 about the supporting shaft, that is, about the opposing direction, via a driving mechanism not illustrated. The light source unit 21 of the present embodiment is swung by the first motor 32 in a range of, for example, ±90 degrees when the downward direction is defined as 0 degrees. The vertical angle adjustment unit 3 is supported, via a supporting shaft not illustrated, to be rotatable with respect to the horizontal angle adjustment unit 4.

The horizontal angle adjustment unit 4 adjusts a horizontal angle, that is, adjusts a pan direction P by causing rotation of the light source unit 21 in the horizontal direction (the front and back direction X and the width direction Y). The horizontal angle adjustment unit 4 includes the base portion 41 and the second motor 42. The main body portion 2 is supported by the base portion 41 to be rotatable in the horizontal direction, via the arm portion 31. The base portion 41 is attached to the ceiling wall 100 and formed in a cubic shape with a longitudinal direction being in the front and back direction. On the base portion 41, a distal end portion 31b of the arm portion 31 in an upper direction side is supported so that the arm portion 31 is supported to be rotatable in the vertical direction Z. The second motor 42 is a horizontal actuator and rotates the arm portion 31 with respect to the base portion 41. The second motor 42 is attached to the base portion 41, and rotates the arm portion 31 about the supporting shaft, that is, the vertical direction, via a driving mechanism not illustrated, to rotate the main body portion 2. The light source unit 21 of the present embodiment is swung by the second motor 42 in a range of, for example, ±180 degrees when any of the horizontal directions is defined as 0 degrees.

In the above description, each actuator is a motor, but the motor is not limited to the actuator, and may be a hydraulic cylinder, a pneumatic cylinder, or the like.

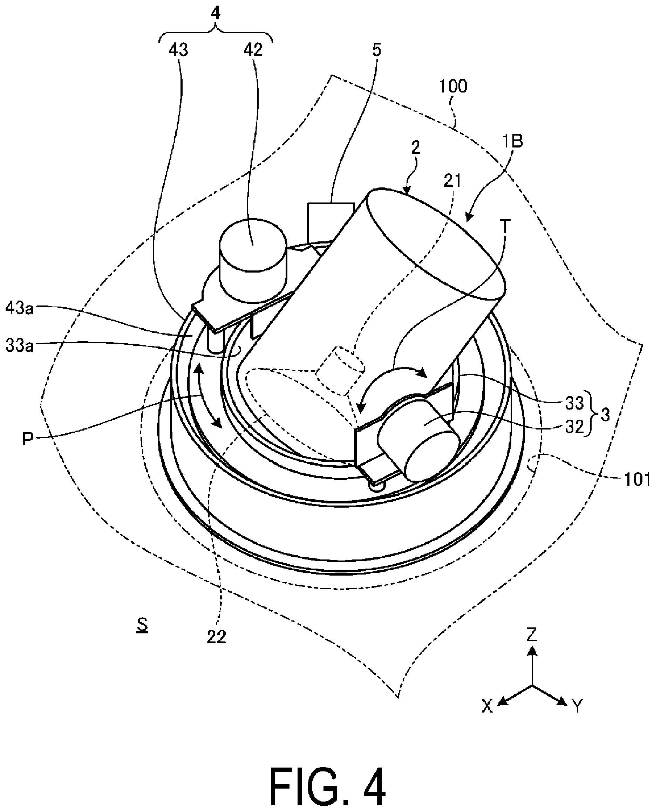

FIG. 4 is a perspective view illustrating an example of a mechanical configuration of another example of the sterilizer 1 (sterilizer 1B). In FIG. 4, the sterilizer 1B differs from the above-described sterilizer 1A in the state of attachment to the ceiling wall 100. Since the basic configuration of the sterilizer 1B is identical or substantially identical to the basic configuration of the sterilizer 1A, description of identical reference numerals will be omitted or simplified.

As illustrated in FIG. 4, the sterilizer 1B irradiates the sterilization target space S with ultraviolet rays UV. The sterilizer 1B is attached to the ceiling wall 100 forming a part of the sterilization target space S. Specifically, the sterilizer 1A is attached to the ceiling wall 100 while protruding from the ceiling wall 100 toward the side opposite to the sterilization target space S side, that is, while being buried in the ceiling wall 100. An opening 101 is formed at the ceiling wall 100, and the sterilizer 1B is attached with the sterilizer 1B, in particular, the opening 22 of the main body portion 2 exposed to the sterilization target space S through the opening 101. The sterilizer 1B includes the main body portion 2 with the light source unit 21 attached, the vertical angle adjustment unit 3, the horizontal angle adjustment unit 4, and the controller 5.

The light source unit 21 irradiating the sterilization target space S with the ultraviolet rays UV in a radiating form is attached to the main body portion 2. The main body portion 2 is formed in a substantially cylindrical shape, with an opening 22 formed at the sterilization target space S side in the axial direction and with the side opposite to the sterilization target space S side closed. The main body portion 2 is supported, via a supporting shaft not illustrated, to be rotatable with respect to the vertical angle adjustment unit 3.

The vertical angle adjustment unit 3 rotates the light source unit 21 in the vertical direction Z, to adjust the vertical angle, that is, a tilt direction T. The vertical angle adjustment unit 3 includes a sub frame portion 33 and the first motor 32. The main body portion 2 is supported by the sub frame portion 33 to be rotatable in the vertical direction Z. The sub frame portion 33, having an internal space 33a formed communicating with the outside through an opening in the upward and downward direction, is formed in a cylindrical shape with the axis in the upward and downward direction. In the internal space 33a of the sub frame portion 33, the main body portion 2 is supported to be rotatable about the horizontal direction. Specifically, in the sub frame portion 33, at least part of the main body portion 2 is accommodated in the internal space 33a, and the main body portion 2 is supported to be rotatable about the vertical direction. The first motor 32 is attached to the sub frame portion 33 and rotates the main body portion 2 about the supporting shaft, that is, about the opposing direction, via a driving mechanism not illustrated. The light source unit 21 of the present embodiment is swung by the first motor 32 in a range of, for example, -35 degrees to +45 degrees when the downward direction is defined as 0 degrees.

The horizontal angle adjustment unit 4 adjusts a horizontal angle, that is, adjusts a pan direction P by causing rotation of the light source unit 21 in the horizontal direction (the front and back direction X and the width direction Y). The horizontal angle adjustment unit 4 includes a base frame portion 43 and the second motor 42. The main body portion 2 is supported by the base frame portion 43 to be rotatable in the horizontal direction, via the sub frame portion 33. The base frame portion 43, with an internal space 43a formed communicating with the outside through an opening in the upward and downward direction, is attached to the ceiling wall 100 while opposing the opening 101 in the upward and downward direction, and is formed in a cylindrical shape with the axis in the upward and downward direction. In the internal space 43a of the base frame portion 43, the main body portion 2 is supported to be rotatable about the vertical direction. Specifically, in the base frame portion 43, at least part of the sub frame portion 33 is accommodated in the internal space 43a, and the sub frame portion 33 is supported to be rotatable about the vertical direction. The light source unit 21 of the present embodiment is swung by the second motor 42 in a range of, for example, +355 degrees when any of the horizontal directions is defined as 0 degrees.

In the above description, each actuator is a motor, but the motor is not limited to the actuator, and may be a hydraulic cylinder, a pneumatic cylinder, or the like.

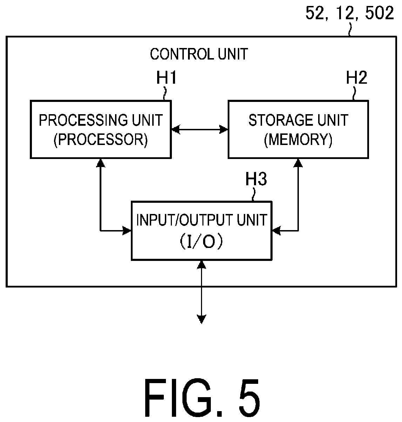

FIG. 5 is a diagram illustrating an example of a hardware configuration of the control unit 52 of the controller 5, the control unit 12 of the terminal 10, or the control unit 502 of the controller 500. In FIG. 5, the control unit 52, 12, 502 has a configuration of a general computer, and includes a processing unit (processor) H1, a storage unit (memory) H2, and an input/output unit (I/O) H3. The storage unit H2 includes a read only memory (ROM), a random access memory (RAM), a nonvolatile-RAM (NV-RAM), a solid state drive (SSD), and a hard disk drive (HDD).

The processing unit H1 executes processing based on data stored in the storage unit H2 or data input from the input/output unit H3 in accordance with a program stored in the storage unit H2. Data obtained by the processing is output from the input/output unit H3.

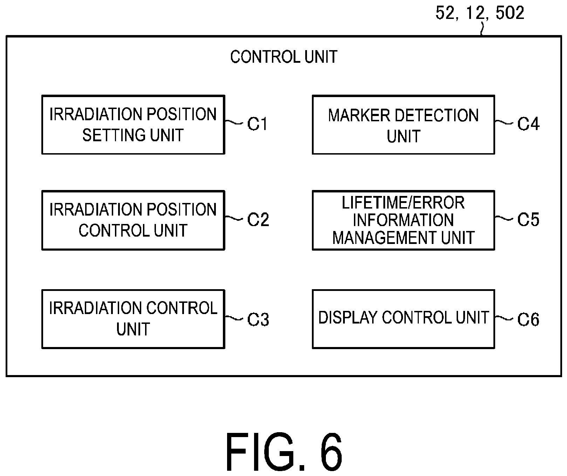

FIG. 6 is a diagram illustrating an example of a functional configuration the control unit 52 of the controller 5, the control unit 12 of the terminal 10, or the control unit 502 of the controller 500. In FIG. 6, the control unit 52, 12, 502 includes an irradiation position setting unit C1, an irradiation position control unit C2, an irradiation control unit C3, a marker detection unit C4, a lifetime/error information management unit C5, and a display control unit C6.

The irradiation position setting unit C1 has a function of setting a position or a range (a range is designated by a plurality of positions or the like) to be irradiated with ultraviolet rays for sterilization. The irradiation position control unit C2 has functions of controlling the vertical angle adjustment unit 3 and the horizontal angle adjustment unit 4 of the sterilizer 1 based on the setting information and controlling the position irradiated with ultraviolet rays. The irradiation control unit C3 has a function of turning ON and OFF the ultraviolet rays, and of changing the intensity of the ultraviolet rays (the intensity of the ultraviolet rays may or may not be changeable depending on the light sources), or changing the wavelengths of the ultraviolet rays (switching can be performed between a wavelength settable for human irradiation and a wavelength not settable for human irradiation but has high sterilization effect) based on the setting information and the like.

The marker detection unit C4 captures the marker signal, based on infrared rays and the like, transmitted from the marker 9 using the detection signal (image signal) from the marker sensor 6, and provides information on a movement direction to the irradiation position control unit C2 to direct the ultraviolet ray irradiation direction to the marker 9 (tracking). When a plurality of the markers 9 are provided, each of the markers 9 can be identified because the pulse pattern varies among the marker signals from the respective markers 9. In this case, the plurality of markers 9 cannot be tracked at once, and thus the markers 9 are sequentially tracked in a predetermined order.

The lifetime/error information management unit C5 has a function of managing the lifetime of the light source unit 21 emitting the ultraviolet rays for sterilization, and error information generated while the sterilizer 1 is operating. The display control unit C6 has a function of displaying the lifetime of the light source unit 21 and the error information on the sterilizer 1 managed by the lifetime/error information management unit C5 using the display unit 8.

### Real Time Operation by User

In FIGS. 1 to 6, the user operates the terminals 10 and the like to change the status of the sterilizer 1 (1A, 1B), the irradiation status of the light source unit 21, and the orientation status of the light source unit 21. For example, when the user operates the terminal 10 to turn ON the light source unit 21, the controller 5 turns ON the light source unit 21 via the light source unit drive circuit 53, based on the irradiation control signal corresponding to the operation information corresponding to the turn ON. With the light source unit 21 turned ON, the sterilizer 1 irradiates the sterilization target space S with the ultraviolet rays UV in a radiating form, to sterilize the air in the sterilization target space S and an object in the sterilization target space S with the ultraviolet rays UV. When the user operates the terminal 10 to change the irradiation range of the ultraviolet rays UV in the sterilization target space S in the vertical direction, the controller 5 drives the first motor 32 via the first motor drive circuit 54 based on the drive control signal for the first motor 32 corresponding to the operation information corresponding to the adjustment of the vertical angle to rotate the light source unit 21 in the vertical direction. When the user operates the terminal 10 to change the irradiation range of the ultraviolet rays UV in the sterilization target space S in the horizontal direction, the controller 5 drives the second motor 42 via the second motor drive circuit 55 based on the drive control signal for the second motor 42 corresponding to the operation information corresponding to the adjustment of the horizontal angle to rotate the light source unit 21 in the horizontal direction. Thus, the user who wants to sterilize an object in the sterilization target space S rotates the light source unit 21 in the vertical direction and the horizontal direction, to move the irradiation range of the ultraviolet rays UV from the light source unit 21 to the object, and performs the sterilization.

As described above, in the sterilizer 1 (1A, 1B) according to the present embodiment, the light source unit 21 can be rotated in the horizontal direction and the vertical direction, so that the irradiation range of the ultraviolet rays UV for the sterilization target space S can be large compared with a case where the light source unit 21 cannot rotate. Thus, a wide range of the sterilization target space S can be sterilized, whereby the environment in the space can be easily improved.

The light source unit 21 emits the ultraviolet rays UV being electromagnetic waves having wavelengths of 190 nm to 230 nm. Thus, even if a person is present in the sterilization target space S and the person is irradiated with the ultraviolet rays UV from the light source unit 21, the impact of the emitted ultraviolet rays on the human body is extremely small. Therefore, even if there is a person in the sterilization target space S, the sterilization by the sterilizer 1 can be safely performed.

In the above description, the sterilizer 1 (1A, 1B) is operated by a manual operation by the user, but is not limited to being operated by the manual operation, and may be operated by automatic operation. For example, a sensor reacting to the movement of an object, such as a human sensor, for example, and having a following mode may be used. Under the following mode, the irradiation range of the ultraviolet rays UV changes to follow the movement of the object. The ultraviolet rays UV may be emitted in the irradiation direction while following a direction of location of a predetermined object, for example, a person. The control unit 52 may have a sterilization mode for an operation set in advance, and in the mode, a floor or a wall forming the sterilization target space S is entirely irradiated with the ultraviolet rays UV for example. The control unit 52 may irradiate the sterilization target space S with the ultraviolet rays UV, during predetermined time, for example, a time zone with absence of any person in the sterilization target space S.

### Setting of Position or Range Automatically Irradiated with Ultraviolet Rays

When the sterilization is to be performed with a desired position or range (designated by a plurality of positions and the like) automatically irradiated with ultraviolet rays, the position or the range needs to be set in advance. The setting of the position or the range needs to be performed by the user, and thus is desired to be easily performable.

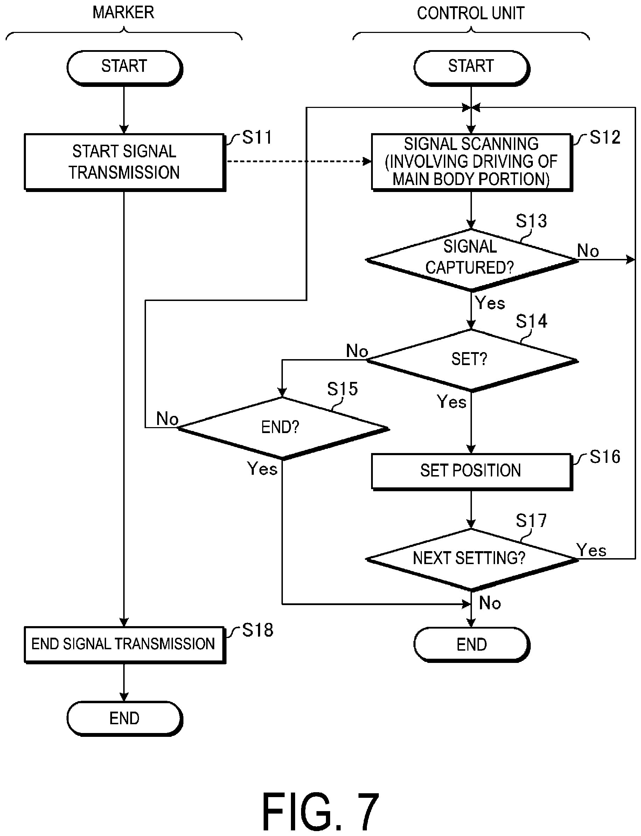

FIG. 7 is a flowchart illustrating an example of control by the control unit 52 of the controller 5, the control unit 12 of the terminal 10, or the control unit 502 of the controller 500, using the marker 9.

In FIG. 7, when the processing starts, transmission of a marker signal starts on the marker 9 side (step S11), and the control unit 52 or the like performs scanning based on a signal from the marker sensor 6 (step S12). For example, the marker sensor 6 covers the entire range in the upward and downward direction (Z-axial direction) but does not cover the entire range in a left and right direction (X-axial direction, Y-axial direction). Therefore, the control unit 52 or the like performs the scanning by rotating the arm portion 31. When the marker sensor 6 is captured as a result of the scanning, the main body portion 2 is also rotated, and the optical axis of the light source unit 21 is directed to the marker sensor 6.

Next, the control unit 52 or the like determines whether the marker signal from the marker 9 has been captured (step S13), and upon determining no capture (No in step S13), repeats the processing from the signal scanning (step S12).

When the marker signal from the marker 9 was determined to have been captured (Yes in step S13), the control unit 52 or the like displays, on the operation/display unit 13, 503, a message or the like asking whether to set a position, to request the user for determination on the setting (step S14).

When the user rejects the setting using the operation/display unit 13, 503 because of a reason such as the user not wanting the captured position to be set (No in step S14), the control unit 52 or the like determines whether the user has issued an instruction to end the setting processing (step S15). When the user was determined to have not issued the instruction to end the setting processing (No in step S15), the control unit 52 or the like repeats the processing from the signal scanning (step S12). When the user was determined to have issued the instruction to end the setting processing (Yes in step S15), the control unit 52 or the like ends the processing.

When the user permits the setting by using the operation/display unit 13, 503 or the like (Yes in Step S14), the control unit 52 or the like sets (stores) the current position (horizontal angle, vertical angle) (Step S16).

Then, the control unit 52 or the like determines whether the next setting has been requested by the user using the operation/display unit 13, 503 or the like (whether the next setting is not rejected) (step S17), and repeats the processing from the signal scanning (step S12) when the request was determined to have been made (no rejection) (Yes in step S17).

When the next setting was determined to have not been requested (rejection) (No in step S17), the control unit 52 or the like ends the processing. The transmission of the marker signal from the marker 9 ends, and also the processing ends (step S18).

### Ultraviolet Ray Visualization Setting

There is no problem as long as the ultraviolet ray irradiation range directed to the marker 9 by the control unit 52 or the like with the sterilizer 1 appropriately capturing the marker 9 is believable. Still, the user may hope to confirm the irradiation range of the ultraviolet ray being truly directed to the marker 9. In this case, since the ultraviolet rays cannot be directly visually confirmed, assistance of some sort is required.

In the present embodiment, the marker 9 is provided with the ultraviolet sensor 92 (FIG. 1) detecting the irradiation of the marker 9 with the ultraviolet rays (the optical axis of the light source unit 21 directed to the marker 9), and the user can be notified of the detection using the operation/display unit 94.

The main body portion 2 is provided with the visible light source unit 7 (FIG. 1) emitting the visible light along the optical axis being the same as the optical axis of the light source unit 21, to enable direct visual recognition of the ultraviolet ray irradiation position by the user based on the visible light irradiation position.

### Simultaneous Setting for Plurality of Positions

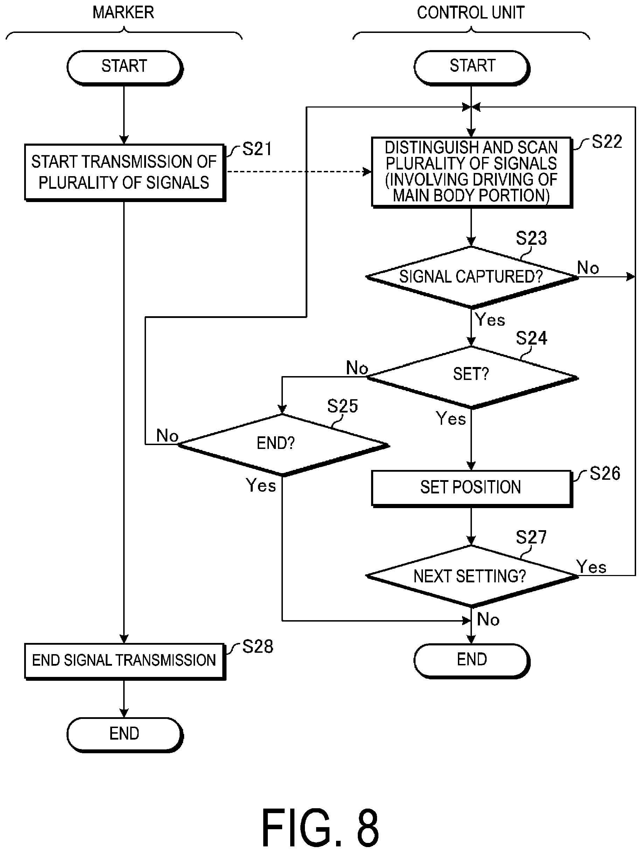

FIG. 8 is a flowchart illustrating another example of control by the control unit 52 of the controller 5, the control unit 12 of the terminal 10, or the control unit 502 of the controller 500, using the marker 9. In FIG. 8, when the processing starts, a plurality of markers 9 start transmitting a plurality of marker signals on the marker 9 side (step S21), and the control unit 52 or the like performs scanning while distinguishing between the plurality of marker signals based on the pulse pattern, using signals from a plurality of marker sensors 6 (step S22).

Next, the control unit 52 or the like determines whether the marker signal from the marker 9 has been captured (step S23). When the capturing was determined to have failed (No in step S23), the processing is repeated from the signal scanning (step S22).

When the marker signal from the marker 9 was determined to have been successfully captured (Yes in step S23), the control unit 52 or the like displays, on the operation/display unit 13, 503, a message or the like asking whether to set a position, to request the user for determination on the setting (step S24).

When the user rejects the setting using the operation/display unit 13, 503 because of a reason such as the user not wanting the captured position to be set (No in step S24), the control unit 52 or the like determines whether the user has issued an instruction to end the setting processing (step S25). When the user was determined to have not issued the instruction to end the setting processing (No in step S25), the control unit 52 or the like repeats the processing from the signal scanning (step S22). When the user was determined to have issued the instruction to end the setting processing (Yes in step S25), the control unit 52 or the like ends the processing.

When the user permits the setting by using the operation/display unit 13, 503 or the like (Yes in Step S24), the control unit 52 or the like directs the optical axis to the captured markers 9 one by one, and sets the positions (horizontal angles, vertical angles) (step S26).

Then, the control unit 52 or the like determines whether the next setting has been requested by the user using the operation/display unit 13, 503 or the like (whether the next setting is not rejected) (step S27), and repeats the processing from the signal scanning (step S22) when the request was determined to have been made (no rejection) (Yes in step S27).

When the next setting was determined to have not been requested (rejection) (No in step S27), the control unit 52 or the like ends the processing. The transmission of the marker signal from the marker 9 ends, and also the processing ends (step S28).

### Automatic Irradiation based on Set Position or Range

Automatic irradiation starts based on a predetermined schedule (time, day of week, date), presence or absence of a person (detection by a human sensor), or the like, and the set position or range is read to be irradiated with the ultraviolet rays. The setting information can include irradiation time common to a plurality of positions or ranges, irradiation time for each individual position or range, irradiation intensity, wavelength, and distance, and the ultraviolet ray irradiation is performed based on the information included in the setting information.

### Lifetime and Error Information Display

Even if the ultraviolet ray irradiation is performed with the position or the range to be sterilized appropriately set, when a predetermined sterilization effect cannot be expected due to the lifetime of the light source unit 21 or due to a normal operation failing to be performed because of a failure or the like of the sterilizer 1, the facility suffers from a huge loss. Therefore, the lifetime of the light source unit 21 and error information on the sterilizer 1 can be easily checked.

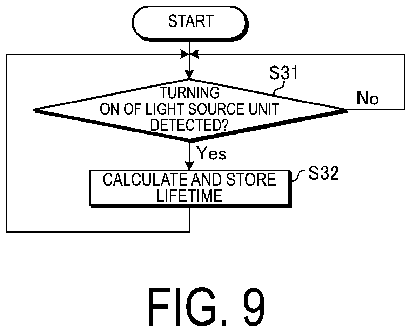

FIG. 9 is a flowchart illustrating an example of processing of calculating lifetime by the control unit 52 of the controller 5, the control unit 12 of the terminal 10, or the control unit 502 of the controller 500, and illustrates an example of processing of acquiring lifetime. In FIG. 9, when the processing starts, the control unit 52 or the like determines whether turning ON of the light source unit 21 is detected (step S31), and repeats the determination upon determining no detection (No in step S31).

When turning ON of the light source unit 21 was determined to be detected (Yes in step S31), the control unit 52 or the like calculates and stores the lifetime (step S32), and returns to the determination on the turning ON (step S31). The lifetime is calculated by subtracting the ON time from the total lifetime (3000 hours for example) of the light source unit 21, for example.

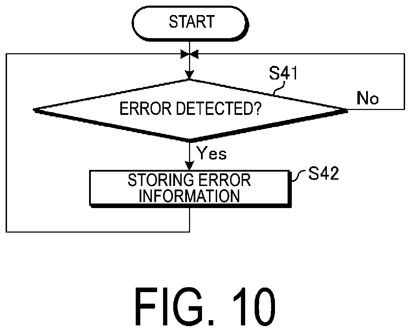

FIG. 10 is a flowchart illustrating an example of processing of acquiring error information by the control unit 52 of the controller 5, the control unit 12 of the terminal 10, or the control unit 502 of the controller 500, and illustrates an example of processing of acquiring error information. In FIG. 10, when the processing starts, the control unit 52 or the like determines whether occurrence of an error in the sterilizer 1 is detected (step S41), and repeats the determination upon determining no detection (No in step S41).

When occurrence of an error in the sterilizer 1 was determined to be detected (Yes in step S41), the control unit 52 or the like stores the error information (step S42), and returns to determination on the error detection (step S41). The error information is, for example, an error code.

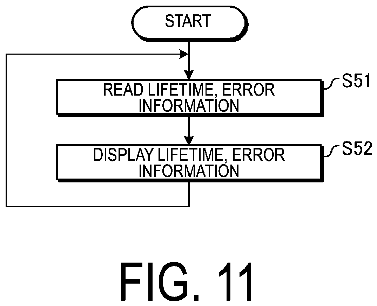

FIG. 11 is a flowchart illustrating an example of processing of displaying lifetime or error information by the control unit 52 of the controller 5, the control unit 12 of the terminal 10, or the control unit 502 of the controller 500, and illustrates an example of processing of displaying lifetime/error information. In FIG. 11, the control unit 52 or the like reads the stored lifetime and/or error information (step S51) and displays the lifetime and/or error information on, for example, the display unit 8 (FIG. 1) (step S52).

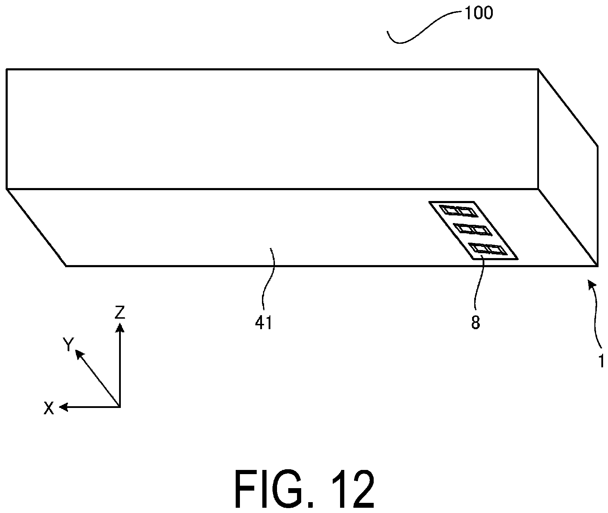

FIG. 12 is a diagram illustrating an example of the display unit 8 displaying the lifetime or the error information. In FIG. 12, the base portion 41 of the sterilizer 1 fixed to the ceiling wall 100 has a lower surface provided with the display unit 8 such as an 8-segment LED or the like, and the display unit 8 displays the lifetime and/or error information.

The embodiment of the present invention has been described above, but the present invention is not limited to the embodiment described above, and various modifications are possible without departing from the spirit of the present invention.

As described above, the sterilization system according to the embodiment includes: a light source unit configured to emit ultraviolet rays in a radiating form; a horizontal angle adjustment unit configured to adjust a horizontal angle of the light source unit by causing rotation of the light source unit in a horizontal direction; and/or a vertical angle adjustment unit configured to adjust a vertical angle of the light source unit by causing rotation of the light source unit in a vertical direction; and a controller configured to receive a marker signal transmitted from a marker via a marker sensor, control rotation of the horizontal angle adjustment unit and/or the vertical angle adjustment unit to direct an optical axis of the light source unit to the marker, and store a horizontal angle and/or a vertical angle of the light source unit in response to a position setting request. Thus, setting for automatic irradiation can be easily performed, and a sterilization target space over a wide range can be easily sterilized.

The marker includes an ultraviolet sensor detecting the ultraviolet rays being emitted with the optical axis of the light source unit directed to the marker. Thus, the position irradiated with ultraviolet rays can be accurately recognized, so that an operation of setting the irradiation target position or range can be accurately performed.

A visible light source emitting visible light in parallel to the ultraviolet rays from the light source unit is included. Thus, the position irradiated with ultraviolet rays can be visually recognized directly, so that an operation of setting the irradiation target position or range can be accurately performed.

A display unit displaying the lifetime of the light source unit and/or error information on the device is included. Thus, a failure to appropriately perform sterilization due to the lifetime of the light source unit or an error in the device can be avoided.

Moreover, the present invention is not limited to the embodiment described above. A configuration obtained by appropriately combining the above-mentioned constituent elements is also included in the present invention. Further effects and modified examples can be easily derived by a person skilled in the art. Thus, a wide range of aspects of the present invention is not limited to the embodiment described above and may be modified variously.

### Reference Signs List

1, 1A, 1B Sterilizer, 21 Light source unit, 3 Vertical angle adjustment unit, 4 Horizontal angle adjustment unit, 5 Controller, 6 Marker sensor, 7 Visible light source unit, 8 Display unit, 9 Marker, 92 Ultraviolet sensor, 10 Terminal, 500 Controller, C1 Irradiation position setting unit, C2 Irradiation position control unit, C3 Irradiation control unit, C4 Marker detection unit, C5 Lifetime/error information management unit, C6 Display control unit

## Claims

1. A sterilization system comprising:
a light source unit configured to emit ultraviolet rays in a radiating form;
a horizontal angle adjustment unit configured to adjust a horizontal angle of the light source unit by causing rotation of the light source unit in a horizontal direction; and/or
a vertical angle adjustment unit configured to adjust a vertical angle of the light source unit by causing rotation of the light source unit in a vertical direction;
and
a controller configured to receive a marker signal transmitted from a marker via a marker sensor, control rotation of the horizontal angle adjustment unit and/or the vertical angle adjustment unit to direct an optical axis of the light source unit to the marker, and store a horizontal angle and/or a vertical angle of the light source unit in response to a position setting request.

2. The sterilization system according to claim 1, wherein
the marker includes an ultraviolet sensor detecting the ultraviolet rays being emitted with the optical axis of the light source unit directed to the marker.

3. The sterilization system according to claim 1 or 2 further comprising:
a visible light source configured to emit visible light in parallel to the ultraviolet rays from the light source unit.

4. The sterilization system according to any one of claims 1 to 3 further comprising:
a display unit configured to display a lifetime of the light source unit and/or error information on a device.

5. A sterilizer comprising:
a light source unit configured to emit ultraviolet rays in a radiating form;
a horizontal angle adjustment unit configured to adjust a horizontal angle of the light source unit by causing rotation of the light source unit in a horizontal direction; and/or
a vertical angle adjustment unit configured to adjust a vertical angle of the light source unit by causing rotation of the light source unit in a vertical direction; and
a controller configured to receive a marker signal transmitted from a marker via a marker sensor, control rotation of the horizontal angle adjustment unit and/or the vertical angle adjustment unit to direct an optical axis of the light source unit to the marker, and store a horizontal angle and/or a vertical angle of the light source unit in response to a position setting request.

6. A controller configured to perform for
a light source unit configured to emit ultraviolet rays in a radiating form, and
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
receiving of a marker signal transmitted from a marker via a marker sensor, controlling of rotation of the horizontal angle adjustment unit and/or the vertical angle adjustment unit to direct an optical axis of the light source unit to the marker, and storing of a horizontal angle and/or a vertical angle of the light source unit in response to a position setting request.

7. A control method comprising, by a computer, executing, for
a light source unit configured to emit ultraviolet rays in a radiating form, and
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
receiving of a marker signal transmitted from a marker via a marker sensor, controlling of rotation of the horizontal angle adjustment unit and/or the vertical angle adjustment unit to direct an optical axis of the light source unit to the marker, and storing of a horizontal angle and/or a vertical angle of the light source unit in response to a position setting request.

8. A control program causing a computer to perform for
a light source unit configured to emit ultraviolet rays in a radiating form, and
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
receiving of a marker signal transmitted from a marker via a marker sensor, controlling of rotation of the horizontal angle adjustment unit and/or the vertical angle adjustment unit to direct an optical axis of the light source unit to the marker, and storing of a horizontal angle and/or a vertical angle of the light source unit in response to a position setting request.
